# EUROPÄISCHE PATENTSCHRIFT

(11) **EP 0 925 796 B1**
(45) Veröffentlichungstag und Bekanntmachung des Hinweises auf die Patenterteilung: **31.03.2004**
(21) Anmeldenummer: 98124159.9
(22) Anmeldetag: 19.12.1998
(51) Int. Cl.: A61M 1/16

(54) **Verfahren und Einrichtung zur Überwachung der Funktionsfähigkeit einer Blutbehandlungsvorrichtung**
Method and means for controlling the functionality of a blood treatment apparatus
Procédé et Dispositif de Surveillance du bon Fonctionnement d'un Appareil de Traitement du Sang

(30) Priorität: 23.12.1997 DE 19757523
(43) Veröffentlichungstag der Anmeldung: 30.06.1999
(73) Patentinhaber: Fresenius Medical Care Deutschland GmbH, 61352 Bad Homburg v.d.H. (DE)
(72) Erfinder: Spickermann, Reiner, 97535 wasserlosen (DE)
(74) Vertreter: Luderschmidt, Schüler & Partner GbR

(56) Entgegenhaltungen:
- DE-A- 4 239 937
- DE-C- 3 923 078

## Beschreibung

Die Erfindung betrifft ein Verfahren zur Überwachung der Funktionsfähigkeit einer Teileinrichtung einer Blutbehandlungsvorrichtung und eine Blutbehandlungsvorrichtung mit einer Einrichtung zur Überwachung der Funktionsfähigkeit einer Teileinrichtung derselben.

Die bekannten Hämodialysegeräte verfügen über eine Kontrolleinrichtung, die es erlaubt, eine bestimmte Ultrafiltrationsrate vorzugeben. Diese Einrichtung sorgt dafür, daß dem Patienten während der Hämodialysebehandlung eine vorgegebene Menge an Ultrafiltrat unabhängig von der Viskosität des zu behandelnden Blutes und den Eigenschaften des Dialysators entzogen wird.

Da ein Defekt in der Einrichtung zur Kontrolle der Ultrafiltration zu einer Gefährdung des Patienten führen kann, verlangen die Sicherheitsnormen, daß ein Schutzsystem vorhanden ist, das eine für den Patienten gefährliche Ultrafiltration ausschließt. Das am häufigsten eingesetzte Schutzsystem überwacht hierzu den Transmembrandruck.

Die Entwicklung von Dialysatoren mit Membranen hoher Permeabilität, sogenannte Highflux-Dialysatoren, hat jedoch dazu geführt, daß mit einer Überwachung des Transmembrandruckes eine zu hohe oder niedrige Ultrafiltrationsrate, die den Patienten gefährden könnte, nicht mit hinreichender Sicherheit erkannt werden kann.

Die DE-A-42 39 937 beschreibt ein Verfahren zur Feststellung der Funktionsfähigkeit einer Teileinrichtung eines Hämodialysegerätes, bei dem während der Dialyse in periodischen Zeitabständen der Dialysator für jeweils ein kurzes Zeitintervall vom Dialysierflüssigkeitskreislauf abgetrennt wird und der Druckverlauf in der Dialysierflüssigkeit im Sinne eines Druckhaltetests auf Abweichung vom stabilen Zustand erfaßt wird. Dieses Verfahren hat sich in der Praxis bewährt. Nachteilig ist jedoch, daß das bekannte Verfahren, bei dem der Dialysator vom Dialysierflüssigkeitskreislauf abgetrennt wird, den Dialysator nicht in die Prüfung mit einbezieht. Defekte O-Ringe an den Dialysatorkupplungen sind aber häufige Ursachen für Leckagen.

Der Erfindung liegt die Aufgabe zugrunde, ein Verfahren zur Überwachung der Funktionsfähigkeit einer Teileinrichtung einer Blutbehandlungsvorrichtung anzugeben, das sowohl den Blutweg als auch den Dialysierflüssigkeitsweg bei der Prüfung mit einschließt. Die Lösung dieser Aufgabe erfolgt erfindungsgemäß mit den im Patentanspruch 1 angegebenen Merkmalen.

Darüber hinaus liegt der Erfindung die Aufgabe zugrunde, eine Blutbehandlungsvorrichtung mit einer Einrichtung zur Überwachung der Funktionsfähigkeit einer Teileinrichtung derselben zu schaffen, die sowohl den Blutweg als auch den Dialysierflüssigkeitsweg bei der Prüfung mit einschließt. Diese Aufgabe wird erfindungsgemäß mit den im Patentanspruch 8 angegebenen Merkmalen gelöst.

Bei dem erfindungsgemäßen Verfahren und der erfindungsgemäßen Vorrichtung wird in einem Teil des Blutweges und des Dialysierflüssigkeitsweges ein Überdruck aufgebaut, der zur Detektion eines Druckabfalls überwacht wird. Der Prüfdruck auf der Dialysierflüssigkeitsseite baut sich infolge des durch die Membran des Dialysators hindurchtretenden Ultrafiltrats auf. Eine eventuelle Leckage in dem abgeschlossenen System, das sowohl einen Teil des Blutweges als auch des Dialysierflüssigkeitsweges umfaßt, wird dann durch einen (erhöhten) Druckabfall detektiert. Die Fehlererkennung kann durch eine Schwellwerterkennung oder eine Kurvenanalyse erfolgen.

Zum Aufbau des Überdurcks wird der Strömungsweg durch die Dialysierflüssigkeitszuführ- und Dialysierflüssigkeitsabführleitung sowie die Blutzuführ- und Blutabführleitung unterbrochen. Sofern die Blutpumpe eine okkludierende Pumpe ist, wird der Strömungsweg durch die Blutzuführleitung mittels der Pumpe unterbrochen. Separate Absperrorgane brauchen somit nicht mehr in der Blutzuführleitung vorgesehen sein. Zum Aufbau des Überdrucks wird die Blutpumpe dann in Betrieb gesetzt. Der Aufbau eines ausreichenden Prüfdrucks kann aktiv über einen Drucksensor überwacht werden oder auch volumengesteuert beispielsweise durch eine festgesetzte Fördermenge der Blutpumpe vollzogen werden. Es ist weiterhin möglich, daß ein ausreichender Prüfdruck dadurch aufgebaut wird, daß die laufende Blutpumpe gleichzeitig mit dem Abtrennen der Blutabführleitung angehalten wird. Der dadurch aufrechterhaltene Systemdruck kann dann als Prüfdruck verwendet werden.

Die zur Erzeugung des Prüfdrucks benötigte Menge an Ultrafiltrat liegt typischerweise bei etwa 10 ml. Da die gesamte Testzeit bei den bekannten Highflux-Dialysatoren unter einer Minute gehalten werden kann, ist ein zyklischer Betrieb beispielsweise in vorgegebenen Zeitintervallen während der Blutbehandlung möglich. Der Druckhaltetest kann aber auch vor der Blutbehandlung während bzw. nach dem Spülvorgang mit Kochsalz durchgeführt werden. Die Initiierung des Druckhaltetest kann von verschiedenen Kriterien abhängig gemacht werden, z.B. nur bei positivem Dialysierflüssigkeitsdruck.

Die dialysatorseitigen Abschnitte der an den Dialysator angeschlossenen Leitungen können durch Absperrorgane getrennt werden. Derartige Absperrorgane sind in den bekannten Dialysegeräten ohnehin vorhanden. So kann die Unterbrechung des Strömungsweges durch die Blutabführleitung beispielsweise mit der venösen Absperrklemme erfolgen. Bei den bekannten Dialysegeräten, die über eine volumetrische Bilanzierkammer verfügen, sind Absperrorgane auch in der Dialysierflüssigkeitszuführ- und -abführleitung vorgesehen. So können die an den Bilanzierkammerhälften der Bilanziereinrichtung eingangs- und ausgangsseitigen Ventile zur Unterbrechung des Strömungsweges eingesetzt werden. Diese wechselseitig geschalteten Ventile erlauben sogar einen Druckhaltetest während der Dialysator von Dialysierflüssigkeit durchströmt wird.

Eine bei diesen Geräten getrennt vorhandene Ultrafiltrationseinrichtung wird angehalten und über den dialysatorseitigen Teil mit auf Dichtigkeit geprüft. Der Vorteil des erfindungsgemäßen Verfahrens bzw. der erfindungsgemäßen Vorrichtung ist, daß sowohl die Überprüfung der Dichtigkeit der Blutleitungen zwischen der Blutpumpe und der venösen Absperrklemme und der Dichtigkeit des Dialysators einschließlich dessen Schlauchkupplungen als auch der Dichtigkeit der Dialysierflüssigkeitsleitungen einschließlich einer eventuell vorhandenen Bilanzier- und Ultrafiltrationseinrichtung möglich ist. Von Vorteil ist auch, daß bei der Dichtigkeitsprüfung gleichzeitig die mechanische Funktion der venösen Absperrklemme sowie die Funktionsfähigkeit der Blutpumpe überprüft wird.

Auf eine mangelnde Funktionsfähigkeit einer Teileinrichtung der Blutbehandlungsvorrichtung wird vorteilhafterweise dann geschlossen, wenn der Druckabfall pro Zeiteinheit größer als ein vorgegebener Grenzwert ist. Es ist aber auch möglich, die Zeitdauer zu messen, in der der Überdruck auf einen bestimmten Wert abfällt. Diese Zeitdauer ist dann mit einem vorgegebenen Grenzwert zu vergleichen. Es ist ebenfalls möglich, die Abweichung der Kurve von vorgegebenen zeitlichen Verläufen auszuwerten.

Vorzugsweise wird der Überdruck in dem dialysatorseitigen Abschnitt der Blutabführleitung überwacht, so daß der venöse Blutdrucksensor, der in den bekannten Dialysegeräten ohnehin vorgesehen ist, eingesetzt werden kann. Im Prinzip kann aber jeder Sensor, der mit dem zu prüfenden Hydraulikvolumen verbunden ist, herangezogen werden. Insofern kann die Überwachung des Prüfdrucks auch mit dialysatseitigen Sensoren erfolgen.

Bei der Detektion eines erhöhten Druckabfalls wird vorzugsweise ein Alarm gegeben. Darüber hinaus können die erforderlichen Sicherheitsvorkehrungen automatisch eingeleitet werden.

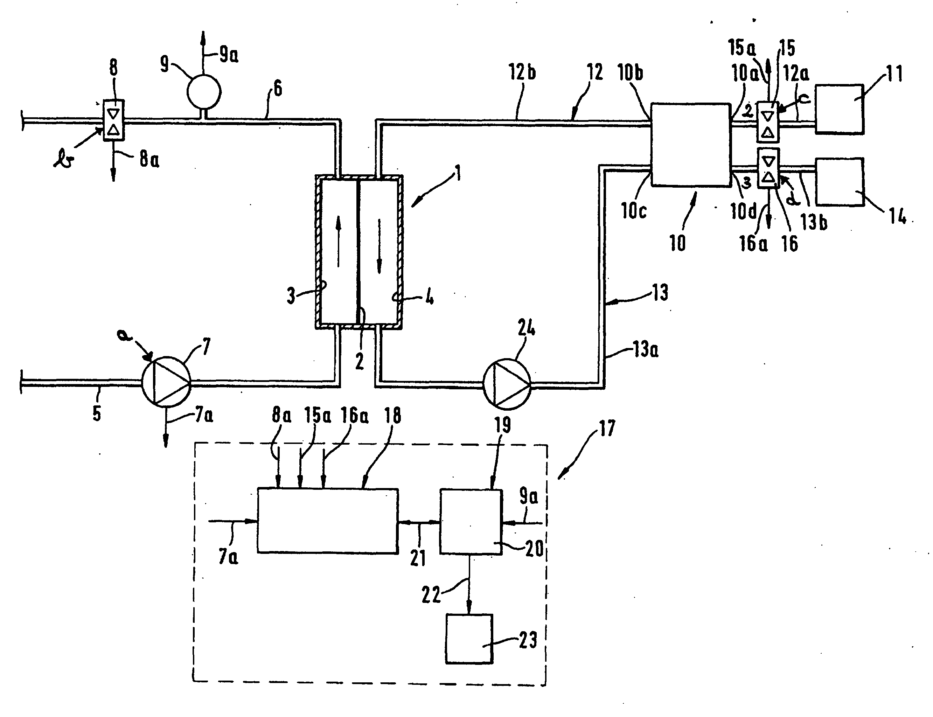

Im folgenden wird ein Ausführungsbeispiel einer Hämodialysevorrichtung mit einer Einrichtung zur Überwachung einer Teileinrichtung derselben unter Bezugnahme auf die Zeichnung näher erläutert, die nur die wesentlichen Komponenten der Hämodialysevorrichtung in vereinfachter schematischer Darstellung zeigt.

Die Hämodialysevorrichtung weist einen Dialysator 1 auf, der durch eine semipermeable Membran 2 in eine Blutkammer 3 und eine Dialysierflüssigkeitskammer 4 unterteilt ist. Eine Blutzuführleitung 5 führt zu dem Einlaß der Blutkammer 3, während eine Blutabführleitung 6 an den Auslaß der Blutkammer 3 angeschlossen ist. In die Blutzuführleitung 5 ist eine okkludierende Blutpumpe 7, z.B. eine Rollenpumpe, geschaltet. Die Rollenpumpe bildet die Trennstelle a. Eine elektromagnetisch betätigbare, venöse Absperrklemme 8 ist an der Trennstelle b in die Blutabführleitung 6 geschaltet. Stromauf der venösen Absperrklemme 8 ist an die Blutabführleitung ein venöser Drucksensor 9 zur Überwachung des Drucks in der Leitung angeschlossen.

Die Hämodialysevorrichtung verfügt über eine Einrichtung 10 zur Bilanzierung der in den Dialysator 1 zufließenden Dialysierflüssigkeit gegen die aus dem Dialysator abfließende Dialysierflüssigkeit sowie zur Entnahme einer vorgegebenen Menge an Ultrafiltrat. Die Figur zeigt die Bilanzier- und Ultrafiltrationseinrichtung 10, die beispielsweise in der DE-A-42 39 937 beschrieben ist, nur andeutungsweise. Die Bilanzier- und Ultrafiltrationseinrichtung ist hier durch ein Ersatzschaltbild mit einem ersten Einlaß 10a und Auslaß 10b für den Anschluß einer Dialysierflüssigkeitszuführleitung und einem zweiten Einlaß 10c und Auslaß 10d für den Anschluß einer Dialysierflüssigkeitsabführleitung dargestellt.

Die Einrichtung zur Bereitstellung der Dialysierflüssigkeit 11 ist über einen ersten Abschnitt 12a der Dialysierflüssigkeitszuführleitung 12 mit dem ersten Einlaß 10a der Bilanzier- und Ultrafiltrationseinrichtung 10 und deren erster Auslaß 10b über einen zweiten Abschnitt 12b der Dialysierflüssigkeitszuführleitung mit dem Einlaß der Dialysierflüssigkeitskammer 4 des Dialysators 1 verbunden. Der Auslaß der Dialysierflüssigkeitskammer 4 des Dialysators 1 ist über einen ersten Abschnitt 13a der Dialysierflüssigkeitsabführleitung 13 mit dem zweiten Einlaß 10c der Bilanzier- und Ultrafiltrationseinrichtung 10 und deren zweiter Auslaß 10d über den zweiten Abschnitt 13b der Dialysierflüssigkeitsabführleitung mit einem Auslaß 14 verbunden. Eine Dialysierflüssigkeitspumpe 24 ist in den ersten Abschnitt 13a der Dialysierflüssigkeitsabführleitung 13 geschaltet.

In den ersten Abschnitt 12a der Dialysierflüssigkeitszuführleitung 12 ist eine zweite elektromagnetisch betätigbare Absperrklemme 15 und in den zweiten Abschnitt 13b der Dialysierflüssigkeitsabführleitung 13 ist eine dritte elektromagnetisch betätigbare Absperrklemme 16 geschaltet. Die zweite und dritte Absperrklemme 15, 16, die an der Trennstelle c stromab der Dialysierflüssigkeitsquelle 11 bzw. an der Trennstelle d stromauf des Auslaufs 14 angeordnet sind, können auch Bestandteil der Bilanzier- und Ultrafiltrationseinrichtung 10 sein. Denn die bekannten Bilanzier- und Ultrafiltrationseinrichtungen, die mit einer volumetrischen Bilanzierkammer arbeiten, verfügen bereits über derartige Absperrorgane, mit denen der Strömungsweg durch die Dialysierflüssigkeitszuführ- und -abführleitung unterbrochen werden kann. Der besseren Übersichtlichkeit wegen sind die zweite und dritte Absperrklemme 15, 16 in der Figur jedoch als von der Bilanzier- und Ultrafiltrationseinrichtung getrennte Bauteile dargestellt.

Die Hämodialysevorrichtung weist eine Einrichtung 17 zur Überwachung der Funktionsfähigkeit von einer Teileinrichtung derselben auf, die eine Steuereinrichtung 18 und eine Druckmeßeinrichtung 19 umfaßt.

Die Steuereinrichtung 18 ist über Steuerleitungen 8a, 15a, 16a mit den Absperrklemmen 8, 15, 16 zur Ansteuerung derselben verbunden. Über eine weitere Steuerleitung 7a ist die Steuereinrichtung 18 mit der Blutpumpe 7 zur Ansteuerung derselben verbunden. Die Meßwerte des venösen Drucksensors 9 werden über eine Datenleitung 9a zu einem Komparator 20 übertragen, der über eine Steuerleitung 21 mit der Steuereinrichtung 18 verbunden ist. Der Komparator 20 ist über eine weitere Steuerleitung 22 mit einem akustischen und/oder optischen Alarmgeber 23 verbunden.

Während des Betriebs der Hämodialysevorrichtung strömt das Blut des Patienten durch die Blutzuführleitung 5 in die Blutkammer 3 des Dialysators 1 und über die Blutabführleitung 6 wieder zurück zum Patienten, während die Dialysierflüssigkeit durch die Dialysierflüssigkeitszuführleitung 12 in die Dialysierflüssigkeitskammer 4 des Dialysators 1 und aus der Dialysierflüssigkeitskammer über die Dialysierflüssigkeitsabführleitung 13 in den Auslauf 14 strömt.

Zur Überprüfung der Funktionsfähigkeit der Teileinrichtung der Hämodialysevorrichtung schließt die Steuereinrichtung 18 zunächst die drei Absperrklemmen 8, 15, 16. Die Blutpumpe 7 wird von der Steuereinrichtung 18 nun solange betrieben, bis sich in dem abgeschlossenen Volumen, das den Dialysator 1 und die dialysatorseitigen Abschnitte der Dialysierflüssigkeitszuführ- und -abführleitung und der Blutzuführ- und -abführleitung umfaßt, ein bestimmter Überdruck aufgebaut hat. Der sich in dem abgeschlossenen Volumen aufbauende Überdruck wird mit dem venösen Drucksensor 9 überwacht und in dem Komparator 20 mit einem vorgegebenen Grenzwert verglichen. Wenn der Überdruck den vorgegebenen Grenzwert erreicht, schaltet die Steuereinrichtung 18 die Blutpumpe 7 ab. Infolge des Überdrucks in der Blutkammer 3 des Dialysators 1 tritt Ultrafiltrat durch dessen Membran 2 in die Dialysierflüssigkeitskammer 4, wodurch der Prüfdruck auf der Dialysatseite erzeugt wird. Der Überdruck wird nun mit dem venösen Drucksensor 9 überwacht, wobei der Druckabfall pro Zeiteinheit erfaßt wird. Nach Ablauf eines bestimmten Zeitintervalls wird der Überdruck mit dem venösen Drucksensor 9 gemessen und das Ausgangssignal des Drucksensors 9 wird in dem Komparator 20 mit einem vorgegebenen Grenzwert verglichen. Wenn der Überdruck unterhalb des Grenzwertes liegt, steuert der Komparator 20 den akustischen und/oder optischen Alarmgeber 23 an, so daß eine Undichtigkeit im System sofort erkannt werden kann und die erforderlichen Sicherheitsvorkehrungen eingeleitet werden können.

Für den Fall, daß der Überdruck nicht unterhalb des vorgegebenen Grenzwertes abfällt, öffnet die Steuereinrichtung 18 wieder die Absperrklemmen 8, 15, 16 und setzt die Blutpumpe 7 wieder in Betrieb. Zum kontrollierten Abbau des Prüfdrucks kann es förderlich sein, die Ultrafiltrationseinrichtung kurz zu aktivieren.

Die Überprüfung der Funktionsfähigkeit kann in bestimmten Zeitintervallen erfolgen. Es ist aber auch nur ein einmaliger Test vor der eigentlichen Dialysebehandlung möglich.

## Patentansprüche

1. Verfahren zur Überwachung der Funktionsfähigkeit einer Teileinrichtung einer Blutbehandlungsvorrichtung, wobei die Teileinrichtung aufweist:
einen durch eine semipermeable Membran (2) in eine erste und eine zweite Kammer (3, 4) unterteilten Dialysator (1),
eine an den Einlaß der ersten Kammer (3) des Dialysators (1) angeschlossene Blutzuführleitung (5), in die eine Blutpumpe (7) geschaltet ist, und eine an den Auslaß der ersten Kammer (3) des Dialysators (1) angeschlossene Blutabführleitung (6),
eine von einer Dialysierflüssigkeitsquelle (11) zu dem Einlaß der zweiten Kammer (4) des Dialysators (1) führende Dialysierflüssigkeitszuführleitung (12) und eine von dem Auslaß der zweiten Kammer (4) des Dialysators (2) zu einem Auslauf (14) führende Dialysierflüssigkeitsabführleitung (13),
mit folgenden Verfahrensschritten:
- Abtrennen eines dialysatorseitigen Abschnitts an einer ersten Trennstelle (c) der Dialysierflüssigkeitszuführleitung (12) von derselben,
- Abtrennen eines dialysatorseitigen Abschnitts an einer zweiten Trennstelle (d) der Dialysierflüssigkeitsabführleitung (13) von derselben,
- Abtrennen eines dialysatorseitigen Abschnitts an einer dritten Trennstelle (b) der Blutabführleitung (6) von derselben,
- Abtrennen eines dialysatorseitigen Abschnitts an einer vierten Trennstelle (a) der Blutzuführleitung (5) von derselben,
- Aufbau eines Überdrucks in dem abgeschlossenen Volumen, das den Dialysator (1) und die dialysatorseitigen Abschnitte der . Dialysierflüssigkeitszuführ- und -abführleitung zwischen der ersten und zweiten Trennstelle und der Blutzuführ- und -abführleitung zwischen der dritten und vierten Trennstelle umfaßt und
- Überwachen des Überdrucks zur Detektion eines Druckabfalls in dem abgeschlossenen Volumen.

2. Verfahren nach Anspruch 1, **dadurch gekennzeichnet, daß** die Blutpumpe (7) eine die vierte Trennstelle bildende okkludierende Pumpe ist, wobei die Blutpumpe nach dem Abtrennen der Dialysierflüssigkeitszuführ- und - abführleitung (12, 13) und der Blutabführleitung (6) zum Aufbau des Überdrucks betrieben wird.

3. Verfahren nach Anspruch 2, **dadurch gekennzeichnet, daß** die Blutpumpe (7) solange betrieben wird, bis sich in dem abgeschlossenen Volumen ein vorbestimmter Überdruck aufgebaut hat.

4. Verfahren nach Anspruch 1, **dadurch gekennzeichnet, daß** die Blutpumpe (7) eine die vierte Trennstelle bildende okkludierende Pumpe ist, wobei die vor dem Abtrennen der Blutabführleitung (6) bereits in Betrieb befindliche Blutpumpe beim Abtrennen der Blutabführleitung angehalten wird.

5. Verfahren nach einem der Ansprüche 1 bis 4, **dadurch gekennzeichnet, daß** auf eine mangelnde Funktionsfähigkeit geschlossen wird, wenn der Druckabfall pro Zeiteinheit größer als ein vorgegebener Grenzwert ist.

6. Verfahren nach einem der Ansprüche 1 bis 5, **dadurch gekennzeichnet, daß** der Überdruck in dem dialysatorseitigen Abschnitt der Blutabführleitung (6) überwacht wird.

7. Verfahren nach einem der Ansprüche 1 bis 6, **dadurch gekennzeichnet, daß** die Dialysierflüssigkeitszuführ- und -abführleitung (12, 13) und die Blutabführleitung (6) unterbrochen werden, wenn die Blutzuführ- und - abführleitung und die erste Kammer (3) des Dialysators (1) während des Betriebes der Blutbehandlungsvorrichtung mit Blut und die Dialysierflüssigkeitszuführ- und -abführleitung und die zweite Kammer (4) des Dialysators (1) mit Dialysierflüssigkeit gefüllt sind.

8. Blutbehandlungsvorrichtung mit einer Teileinrichtung, wobei die Teileinrichtung aufweist:
einen durch eine semipermeable Membran (2) in eine erste und eine zweite Kammer (3, 4) unterteilten Dialysator (1),
eine an den Einlaß der ersten Kammer (3) des Dialysators (1) angeschlossene Blutzuführleitung (5), in die eine Blutpumpe (7) geschaltet ist, und eine an den Auslaß der ersten Kammer (3) des Dialysators (1) angeschlossene Blutabführleitung (6), in die ein erstes Absperrorgan (8) geschaltet ist,
eine von einer Dialyiserflüssigkeitsquelle (11) zu dem Einlaß der zweiten Kammer (4) des Dialysators (1) führende Dialysierflüssigkeitszuführleitung (12), in die ein zweites Absperrorgan (15) geschaltet ist, und eine von dem Auslaß der zweiten Kammer (4) des Dialysators (1) zu einem Auslauf (14) führende
Dialysierflüssigkeitsabführleitung (13), in die ein drittes Absperrorgan (16) geschaltet ist,
**gekennzeichnet durch**,
eine Einrichtung (17) zur Überwachung der Funktionsfähigkeit der Teileinrichtung, wobei die Überwachungseinrichtung umfaßt:
eine Steuereinrichtung (18), die die Absperrorgane und die Blutpumpe derart austeuern kann, daß zum Aufbau eines Überdrucks in dem abgeschlossenen Volumen, das den Dialysator (1) und die dialysatorseitigen Abschnitte der Blutzuführ- und -abführleitung (5, 6) zwischen dem ersten Absperrorgan (8) und der Blutpumpe (7) und die dialysatorseitigen Abschnitte der Dialysierflüssigkeitszuführ- und -abführleitung (12, 13) zwischen dem zweiten und dritten Absperrorgan (15, 16) umfaßt, die Absperrorgane (8, 15, 16) absperrbar und die Blutpumpe (7) betreibbar ist, und
eine Druckmeßeinrichtung zur Detektion eines Druckabfalls in dem abgeschlossenen Volumen.

9. Vorrichtung nach Anspruch 8, **dadurch gekennzeichnet, daß** die Druckmeßeinrichtung (19) einen Drucksensor (9) und einen Komparator (20) zum Vergleichen des Ausgangssignals des Drucksensors mit einem vorgegebenen Grenzwert aufweist.

10. Vorrichtung nach Anspruch 9, **dadurch gekennzeichnet, daß** der Drucksensor (9) in der Blutabführleitung (6) zwischen der ersten Kammer (3) des Dialysators (1) und dem ersten Absperrorgan (8) angeordnet ist.

11. Vorrichtung nach einem der Ansprüche 8 bis 10, **dadurch gekennzeichnet, daß** die Überwachungseinrichtung (17) einen Alarmgeber (23) zur Abgabe eines akustischen und/oder optischen Alarms bei der Detektion eines Druckabfalls in dem abgeschlossenen Volumen aufweist.

## Claims

1. Method for controlling the functionality of a sub-device of a blood treatment apparatus, wherein the sub-device exhibits:
a dialyser (1) subdivided by a semipermeable membrane (2) into a first and second chamber (3, 4),
a blood feed line (5) which is connected to the inlet of the first chamber (3) of the dialyser (1) and in which a blood pump (7) is inserted, and a blood discharge line (6) connected to the outlet of the first chamber (3) of the dialyser (1).
a dialysis fluid feed line (12) leading from a dialysis fluid source (11) to the inlet of the second chamber (4) of the dialyser (1), and a dialysis fluid discharge line (13) leading from the outlet of the second chamber (4) of the dialyser (2) to an outlet (14),
with the following process stages:
- separation of a section on the dialyser side at a first spearating point (c) of the dialysis fluid feed line (12) of the same,
- separation of a section on the dialyser side at a second separating point (d) of the dialysis fluid discharge line (13) of the same,
- separation of a section on the dialyser side at a third separating point (b) of the blood discharge line (6) of the same.
- separation of a section on the dialyser side at a fourth separating point (a) of the blood feed line (5) of the same,
- development of an excess pressure in the sealed volume which comprises the dialyser (1) and the sections on me dialyser side of the dialysis fluid feed and discharge line between the first and second separating points, and of the blood feed and discharge line between the third and fourth separating points, and
- monitoring of the excess pressure for detecting a reduction in pressure in the sealed volume.

2. Method according to claim 1, **characterised in that** the blood pump (7) is an occluding pump forming the fourth separating point, wherein the blood pump is operated to develop the excess pressure after separation of the dialysis fluid feed and discharge line (12, 13) and the blood discharge line (6).

3. Method according to claim 2, **characterised in that** the blood pump (7) is operated until a predetermined excess pressure has developed in the sealed volume.

4. Method according to claim 1, **characterised in that** the blood pump (7) is an occluding pump forming the fourth separating point, wherein the blood pump already in operation before separation of the blood discharge line (6) is stopped when the blood discharge line is separated.

5. Method according to one of claims 1 to 4, **characterised in that** a lack of functionality is detected if the reduction in pressure per unit of time is greater than a predetermined limit value.

6. Method according to one of claims 1 to 5, **characterised in that** the excess pressure in the section of the blood discharge line (6) on the dialyser side is monitored.

7. Method according to one of claims 1 to 6, **characterised in that** the dialysis fluid feed and discharge line (12, 13) and the blood discharge fine (6) are interrupted when the blood feed and discharge line and the first chamber (3) of the dialyser (1) are filled with blood during operation of the blood treatment apparatus, and when the dialysis fluid feed and discharge line and the second chamber (4) of the dialyser (1) are filled with dialysis fluid.

8. Blood treatment apparatus with a sub-device, wherein the sub-device exhibits:
a dialyser (1) subdivided by a semipermeable membrane (2) into a first and second chamber (3, 4),
a blood feed pipe (5) which is connected to the inlet of the first chamber (3) of the dialyser (1) and in which a blood pump (7) is inserted, and a blood discharge fine which is connected to the outlet of the first chamber (3) of the dialyser (1) and in which a first shutoff mechanism (8) is inserted,
a dialysis fluid feed line (12) which leads from a dialysis fluid source (11) to the inlet of the second chamber (4) of the dialyser (1) and in which a second shutoff mechanism (15) is inserted, and a dialysis fluid discharge line (13) which leads from the outlet of the second chamber (4) of the dialyser (1) to an outlet (14) and in which a third shutoff mechanisms (16) is inserted.
**characterised by**
a device (17) for monitoring the functionality of the sub-device, wherein the monitoring device comprises:
a control device (18) which is able to activate the shutoff mechanisms and blood pump so that in order to develop an excess pressure the shutoff mechanisms (8, 15, 16) can be shut off and the blood pump (7) operated in the volume which comprises the dialyser (1) and the sections of the blood feed and discharge line (5, 6) on the dialyser side between the first shutoff mechanism (8) and the blood pump (7) and the sections of the dialysis fluid feed and discharge line (12, 13) on the dialyser side between the second and third shutoff mechanism (15, 16), and
a pressure measuring device for detecting a reduction in pressure in the sealed volume.

9. Apparatus according to claim 8, **characterised in that** the pressure measuring device (19) exhibits a pressure sensor (9) and a comparator (20) for comparing the output signal of the pressure sensor with a predetermined limit value.

10. Apparatus according to claim 9, **characterised in that** the pressure sensor (9) is arranged in the blood discharge line (6) between the first chamber (3) of the dialyser (1) and the first shutoff mechanism (8).

11. Apparatus according to one of claims 8 to 10, **characterised in that** the monitoring device (17) exhibits an alarm transmitter (23) for emitting an acoustic and/or optical alarm when a reduction in pressure in the sealed volume is detected.

## Revendications

1. Procédé pour surveiller la capacité de fonctionnement d'un dispositif partiel d'un dispositif de traitement du sang, où le dispositif partiel comprend :
un dialyseur (1) divisé par une membrane semi-perméable (2) en une première chambre et une deuxième chambre (3, 4),
une conduite d'amenée de sang (5) raccordée à l'entrée de la première chambre (3) du dialyseur (1), dans laquelle est branchée une pompe à sang (7), et une conduite d'évacuation de sang (6) raccordée à la sortie de la première chambre (3) du dialyseur (1),
une conduite d'amenée de liquide de dialyse (12) menant d'une source de liquide de dialyse (11) à l'entrée de la deuxième chambre (4) du dialyseur (1) et une conduite d'évacuation de liquide de dialyse (13) menant de la sortie de la deuxième chambre (4) du dialyseur (2) à un dispositif d'écoulement (14),
avec les étapes de procédé suivantes :
- séparation d'une section du côté du dialyseur au niveau d'un premier point de séparation (c) de la conduite d'amenée de liquide de dialyse (12),
- séparation d'une section du côté du dialyseur au niveau d'un deuxième point de séparation (d) de la conduite d'évacuation de liquide de dialyse (13),
- séparation d'une section du côté du dialyseur au niveau d'un troisième point de séparation (b) de la conduite d'évacuation de sang (6),
- séparation d'une section du côté du dialyseur au niveau d'un quatrième point de séparation (a) de la conduite d'évacuation de sang (5),
- formation d'une surpression dans le volume fermé qui comprend le dialyseur (1) et les sections du côté du dialyseur de la conduite d'amenée et de la conduite d'évacuation de liquide de dialyse entre le premier et le deuxième point de séparation et de la conduite d'amenée et de la conduite d'évacuation de sang entre les troisième et quatrième points de séparation et
- surveillance de la surpression pour la détection d'une chute de pression dans le volume fermé.

2. Procédé selon la revendication 1 **caractérisé en ce que** la pompe à sang (7) est une pompe d'occlusion formant le quatrième point de séparation, où la pompe à sang est mise en marche pour la formation de la surpression après la séparation de la conduite d'amenée et de la conduite d'évacuation de liquide de dialyse (12, 13) et de la conduite d'évacuation de sang (6).

3. Procédé selon la revendication 2 **caractérisé en ce que** la pompe à sang (7) est amenée à fonctionner jusqu'à ce qu'une surpression prédéterminée soit apparue dans le volume fermé.

4. Procédé selon la revendication 1 **caractérisé en ce que** la pompe à sang (7) est une pompe d'occlusion formant le quatrième point de séparation, où la pompe à sang qui est déjà en fonctionnement avant la séparation de la conduite d'évacuation de sang (6) est arrêtée lors de la séparation de la conduite d'évacuation de sang.

5. Procédé selon l'une des revendications 1 à 4 **caractérisé en ce que** l'on conclut à une capacité de fonctionnement insuffisante quand la chute de pression par unité de temps est supérieure à une valeur limite prédéterminée.

6. Procédé selon l'une des revendications 1 à 5 **caractérisé en ce que** la surpression est surveillée dans la section du côté du dialyseur de la conduite d'évacuation de sang (6).

7. Procédé selon l'une des revendications 1 à 6 **caractérisé en ce que** la conduite d'amenée et la conduite d'évacuation de liquide de dialyse (12, 13) et la conduite d'évacuation de sang (6) sont interrompues quand la conduite d'amenée et la conduite d'évacuation de sang et la première chambre (3) du dialyseur (1) sont remplies de sang et la conduite d'amenée et la conduite d'évacuation de liquide de dialyse et la deuxième chambre (4) du dialyseur (1) sont remplies de liquide de dialyse pendant le fonctionnement du dispositif de traitement du sang.

8. Dispositif de traitement du sang comportant un dispositif partiel, où le dispositif partiel comprend :
un dialyseur (1) divisé par une membrane semi-perméable (2) en une première et une seconde chambre (3, 4),
une conduite d'amenée de sang (5) raccordée à l'entrée de la première chambre (3) du dialyseur (1), dans laquelle est branchée une pompe à sang (7), et une conduite d'évacuation de sang (6) raccordée à la sortie de la première chambre (3) du dialyseur (1), dans laquelle est branché un premier organe d'arrêt (8),
une conduite d'amenée de liquide de dialyse (12) menant d'une source de liquide de dialyse (11) à l'entrée de la deuxième chambre (4) du dialyseur (1), dans laquelle est branché un deuxième organe d'arrêt (15), et une conduite d'évacuation de liquide de dialyse (13) menant de la sortie de la deuxième chambre (4) du dialyseur (1) à un dispositif d'écoulement (14), dans laquelle est branché un troisième organe d'arrêt (16),
**caractérisé par** un dispositif (17) pour surveiller la capacité de fonctionnement du dispositif partiel, où le dispositif de surveillance comprend :
un dispositif de commande (18) qui peut commander les organes d'arrêt et la pompe à sang de telle manière que les organes d'arrêt (8, 15, 16) peuvent être fermés et la pompe à sang (7) peut être mise en marche pour la formation d'une surpression dans le volume fermé qui comprend le dialyseur (1) et les sections du côté du dialyseur de la conduite d'amenée et de la conduite d'évacuation de sang (5, 6) entre le premier organe d'arrêt (8) et la pompe à sang (7) et les sections du côté du dialyseur de la conduite d'amenée et de la conduite d'évacuation de liquide de dialyse (12, 13) entre le deuxième et le troisième organe d'arrêt (15, 16), et
un dispositif de mesure de la pression pour la détection d'une chute de pression dans le volume fermé.

9. Dispositif selon la revendication 8 **caractérisé en ce que** le dispositif de mesure de la pression (19) comporte un capteur de pression (9) et un comparateur (20) pour comparer le signal de sortie du capteur de pression avec une valeur limite prédéterminée.

10. Dispositif selon la revendication 9 **caractérisé en ce que** le capteur de pression (9) est disposé dans la conduite d'évacuation de sang (6) entre la première chambre (3) du dialyseur (1) et le premier organe d'arrêt (8).

11. Dispositif selon l'une des revendications 8 à 10 **caractérisé en ce que** le dispositif de surveillance (17) comporte un système d'alarme (23) pour délivrer une alarme acoustique et/ou optique lors de la détection d'une chute de pression dans le volume fermé.
